Europäisches Patentamt

European Patent Office

Office européen des brevets

(11) Veröffentlichungsnummer: **0 358 008**

**A1**

(12) # EUROPÄISCHE PATENTANMELDUNG

(21) Anmeldenummer: 89115121.9

(22) Anmeldetag: 16.08.89

(51) Int. Cl.5: **A61K 31/505** , **A61K 31/40** ,
//(A61K31/505,31:40)

Ein Antrag gemäss Regel 88 EPÜ auf Berichtigung der Beschreibung (Seiten 4 und 8) liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: 15.08.88 BG 85235/88

(43) Veröffentlichungstag der Anmeldung:
**14.03.90 Patentblatt 90/11**

(84) Benannte Vertragsstaaten:
**AT BE CH DE ES FR GB LI NL SE**

(71) Anmelder: **RNPISMP " N.I. PIROGOV"**
**Boul. Totleben 21**
**BG-1606 Sofia(BG)**

(72) Erfinder: **Alexandrov, Nikola Georgiev, Dr. med.**
**Komplex Mladost Bl. 335-A**
**Sofia(BG)**
Erfinder: **Vanev, Manol Tzenev, Dr. med.**
**Boul. Bulgaria Nr. 14**
**Sofia(BG)**
Erfinder: **Nikolova, Milka Petrova**
**Rayko Daskalov Strasse Nr. 3**
**Sofia(BG)**
Erfinder: **Ogneva, Vesselina Koleva, Dr. med.**
**Anton Ivanov Strasse Nr. 122**
**Sofia(BG)**
Erfinder: **Vitkova, Snejana Georgieva**
**N. Kamenov Strasse Bl. 252/4/76**
**Sofia(BG)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Ernährungstherapeutisches Mittel.**

(57) Beschrieben wird ein ernährungstherapeutisches Mittel, das in der medizinischen Praxis zur Behandlung von Patienten verwendet werden kann, die parenteral oder enteral ernährt werden müssen, wie zum Beispiel bei alten Menschen und kleinen Kindern mit spezifischer Ernährungsweise.

Das erfindungsgemäße Mittel verbessert die Ernährungstherapie, beseitigt die unerwünschten Nebeneffekte der bekannten Mittel und verbessert die Resorption für Aminosäuren, Kohlehydraten und besonders für Fette. Ausgeprägt ist seine synergistische Wirkung. Das Mittel kann bei Schwerkranken angewandt werden, bei denen die einzige Ernährungsart die parenterale ist. Außerdem kann es zur raschen Genesung nach schweren Erkrankungen bei Kindern und alten Menschen und in Fällen angewandt werden, in denen es bei geistiger oder physischer Belastung zu einem erhöhten Energieverbrauch kommt.

Das Mittel enthält Fette, Eiweiße und/oder Kohlehydrate, und nootrope Stoffe, und zwar Pyracetam (2-Oxo-1-pyrrolidinacetamid), Orotsäure oder ein Gemisch beider Stoffe in einem Gewichtsverhältnis des Nahrungsgemisches zum nootropen Stoff von 10:1,1 bis 10:0,05. Der nootrope Stoff ist vorzugsweise eine Kombination von Pyracetam und Orotsäure in einem Verhältnis von 20:1.

## Ernährungstherapeutisches Mittel

Die Erfindung betrifft ein ernährungstherapeutisches Mittel, das in der medizinischen Praxis zur Behandlung von Patienten verwendet werden kann, die parenteral oder enteral ernährt werden müssen, wie zum Beispiel bei alten Menschen und kleinen Kindern mit spezifischer Ernährungsweise (Diät).

Bekannt sind ernährungstherapeutische Mittel auf der Basis von Kohlehydraten, Eiweißen und Fetten. Bekannt ist zum Beispiel eine intravenös zu verabreichende Fettemulsion, die Buttersäureglyceride, Ei-Phosphate, Glycerin in wäßriger Lösung und Natronlauge zur Einstellung des neutralen pH enthält und einen Brennwert von 1,1 cal (4,601 J) pro ml Lösung aufweist (A.S. Prasad, Current Therapy, 1982, 460).

Bekannt ist auch ein ernährungstherapeutisches Mittel, das eine kristalline nichtpyrogene Lösung von Aminosäuren und eines Elektrolyten für intravenöse Infusionen darstellt, das ebenfalls für die parenterale Ernährung von Kranken verwendet werden kann ( A.S. Prasad, Current Therapy, 1982, 460).

Die Nachteile der bekannten ernährungstherapeutischen Mittel bestehen in der Schwierigkeit der Resorption der Aminosäuren und der Kohlehydrate und besonders der Fette durch den Orga nismus und im Auftreten von Nebenerscheinungen, die besonders stark bei Patienten nach schweren Operationen, Verletzungen und Schocks zutage treten, wenn die Patienten gleichzeitig einer Ernährung mit besonders hohem Brennwert bedürfen. Diese unerwünschten Nebenerscheinungen können früh zutage treten oder Spätfolgen darstellen. Zu den früh auftretenden Nebenerscheinungen zählen Schwindelgefühl, Kopfschmerzen, Schläfrigkeit, Temperaturerhöhung, Brust- und Rückenschmerzen, Ekelgefühl, Brechreiz und Hyperlipämie, und zu den Spätfolgen die Folgen aufgrund der Fettablagerung in der Leber, Vergrößerung von Leber und Milz, Symptome von Gelbsucht, Trombozytopenie, Leukopenie u.a.

Bekannt sind die nootropen Mittel Pyracetam, das 2-Oxo-Pyrolidinacetamid darstellt und zur Behandlung verschiedener Erkrankungen, die mit hypoxischen ischämischen und dismetabolischen Zuständen des Gehirns, Gedächtnisstörungen und verminderter Merkfähigkeit verbunden sind, verwendet werden kann (US-PA Nr. 3 459 738), die Orotsäure, welche die Uracil-4-carbonsäure bzw. das Vitamin $B_{13}$ darstellt und eine hepatoprotektive and antihypoxische Wirkung auf das Leberparenchym sowie teilweise auf das ZNS ausübt (US-PA Nr. 2 937 175) und das Präparat Orocetam, das ein synergistische Gemisch aus Pyracetam und Orotsäure darstellt (BL-Urheberschein Nr. 70 004 = EP-PA 87 101 630 vom 6.2.1987).

Nootrope Mittel wurden bisher nicht zu ernährungstherapeutischen Zwecken verwendet.

Die Aufgabe der Erfindung besteht in der Bereitstellung eines ernährungstherapeutischen Mittels, das zu einer Verbesserung der Resorption der Aminosäuren, Fette und Kohlehydrate im Organismus führt und geringere Nebenwirkungen aufweist.

Die Aufgabe wurde durch ein ernährungstherapeutisches Mittel auf der Basis von Fetten, Eiweißen und/oder Kohlehydraten ge löst, das nootrope Stoffe enthält, und zwar Pyracetam (2-Oxo-1-pyrolidin-acetamid), Orotsäure oder ein Gemisch beider Stoffe in einem Gewichtsverhältnis des Nahrungsgemisches zu den nootropen Stoffen von 10:1,1 bis 10:0,05.

Der nootrope Stoff stellt vorzugsweise ein Gemisch aus Pyracetam und Orotsäure in einem Verhältnis von 20:1 dar.

Es wurde festgestellt, daß bei kombinierter Anwendung von Aminosäurelösung, Fettemulsion und dem nootropen Mittel Pyracetam oder Orotsäure oder einem entsprechenden Gemisch sich eine Verringerung des Lipidspiegels im Organismus und eine Wiederherstellung der Hauptstrukturelemente biologischer Membranen sowie eine Verbesserung der Resorption für Aminosäuren und Kohlehydrate in den Leberzellen feststellen läßt, was zu einer Wiederherstellung der Ultrastruktur der Hepatozyten und anderer Hauptstrukturelemente führt.

Die stärkste potenzierende Wirkung zeigt sich in einer Verringerung des Lipidspiegels in den Leberzellen und in einer Wiederherstellung ihrer Ultrastruktur. Darin zeigt sich die potenzierende Wirkung des nootropen Mittels auf die Resorption der Aminosäuren, Kohlehydrate und insbesondere der Fette.

Das erfindungsgemäße Mittel kann peroral oder vorzugsweise bei Schwerkranken intravenös angewandt werden. So zum Beispiel wird eine 10 %-ige Lösung einer Fettemulsion (Liposin) in einer Menge von 500 ml täglich tropfenweise und 4 bis 12 g Pyracetam und/oder 0,2 bis 0,6 g Orotsäure oder ein Gemisch von beiden in den angegebenen Mengen intravenös verabreicht.

Das erfindungsgemäße Mittel wurde auch als Behandlungsmethode bei 4 Patienten angewandt, und zwar jeweils dem Zustand entsprechend nach subtotaler Gastrektomie, nach korrodierender Verätzung der Speiseröhre durch Salzsäure, bei vollständiger Stenose der Speiseröhre nach korrodierender Verätzung durch Natronlauge sowie nach subtotaler Dünndarmresektion. Den Patienten wurde eine Infusion aus Fettemulsion zusammen mit einem Gemisch aus Pyracetam und Orotsäure (Orocetam) verabreicht. Die entsprechenden Testergebnisse sind in den die Erfindung illustrierenden Beispielen angegeben.

Die Vorteile des erfindungsgemäßen Mittels bestehen darin, daß die Ernährungstherapie verbessert wird, die unerwünschten Nebeneffekte der bekannten Mittel beseitigt werden und die Resorption für Aminosäuren, Kohlehydrate und besonders für Fette verbessert wird, sodaß es bei Schwerkranken angewandt werden kann, bei denen die einzige Ernährungsart die parenterale ist. Außerdem kann es zur raschen Genesung nach schweren Erkrankungen bei Kindern und alte Menschen und in Fällen angewandt werden, in denen es bei geistiger oder physischer Belastung zu einem erhöhten Energieverbrauch kommt.

Die Erfindung wird durch die nachfolgenden Ausführungsbeispiele näher erläutert:

Beispiel 1

Pharmakologische Untersuchungen des Lipidspiegels im Lebergewebe durch Anfärbung und mit Hilfe eines Lichtmikrosops

Untersucht wurden 14 Hunde der Rasse "Beagle", von denen 4 als Kontrolltiere und 8 als Versuchstiere verwendet wurden. Letztere wurden in zwei Gruppen eingeteilt. Im Verlaufe von 8 Tagen wurden 4 Tiere täglich mit einer Fettemulsion (Liposin) bei einer Dosierung von 6,2 ml/kg Körpergewicht behandelt. Die übrigen 4 Tiere wurden nach derselben Zeit mit Fettemulsion (Liposin) bei derselben Dosis und mit einem Gemisch von Pyracetam und Orotsäure (Orocetam) in einer Menge von 1,3 ml/kg Körpergewicht behandelt. Am 8. Tag wurden die Versuchs-und Kontrolltiere abgetötet. Stücke der Leber der Tiere wurden isoliert und in neutralem Formalin (Formol) für histochemische Untersuchungen fixiert. Andere Organstücke wurden in einer Lösung von Glutaraldehyd unter nachfolgender Fixierung in $OSO_4^-$ für elektronenmikroskopische Untersuchungen fixiert.

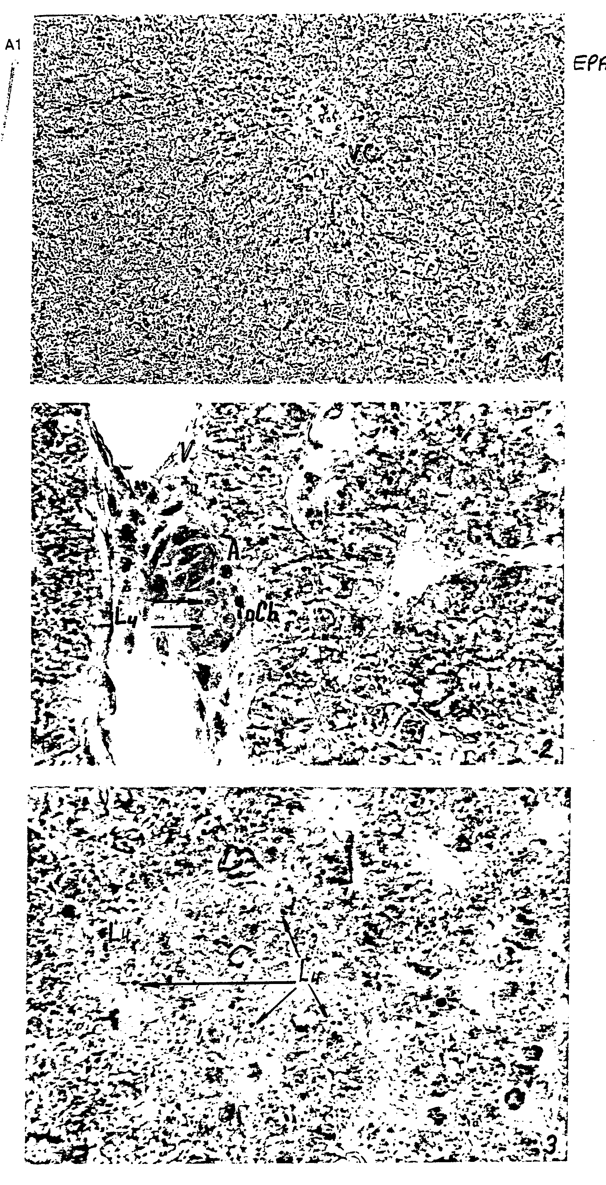

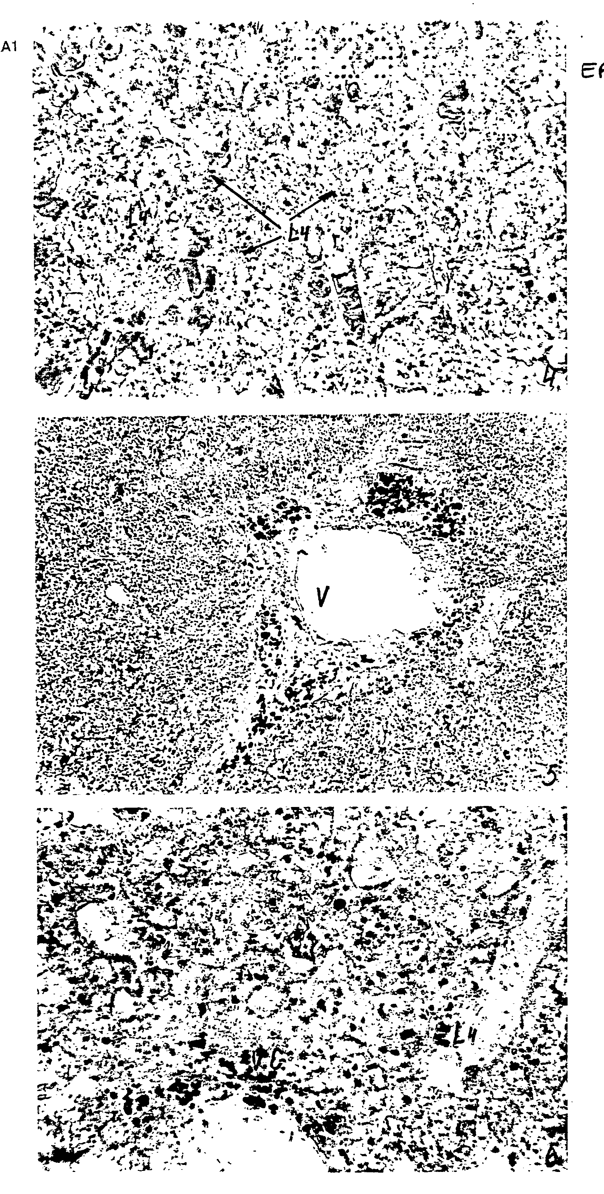

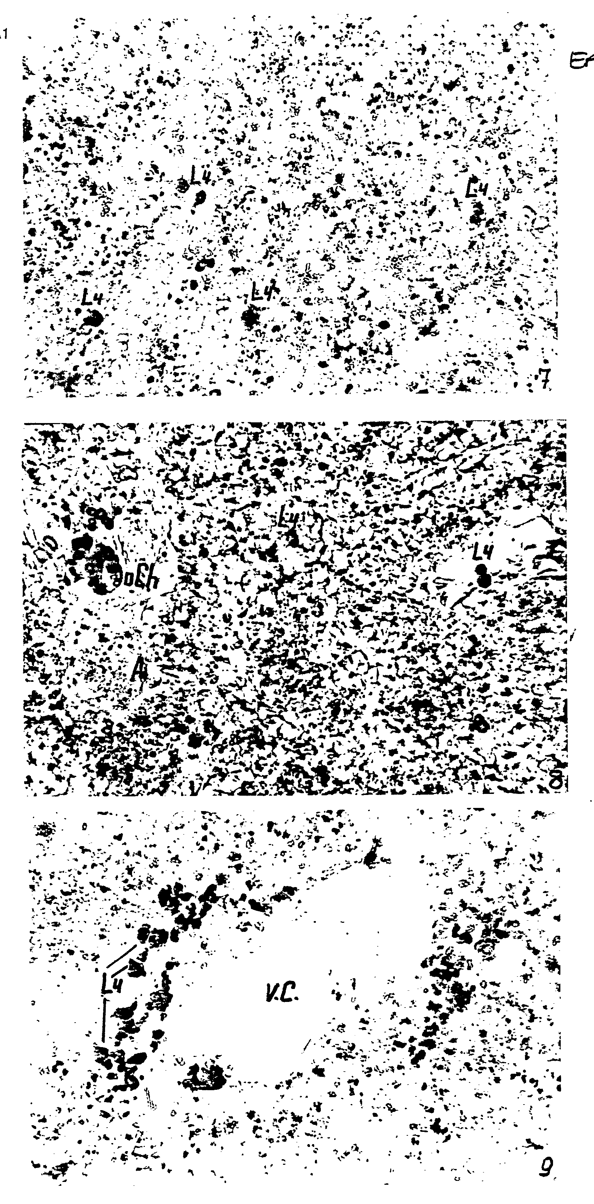

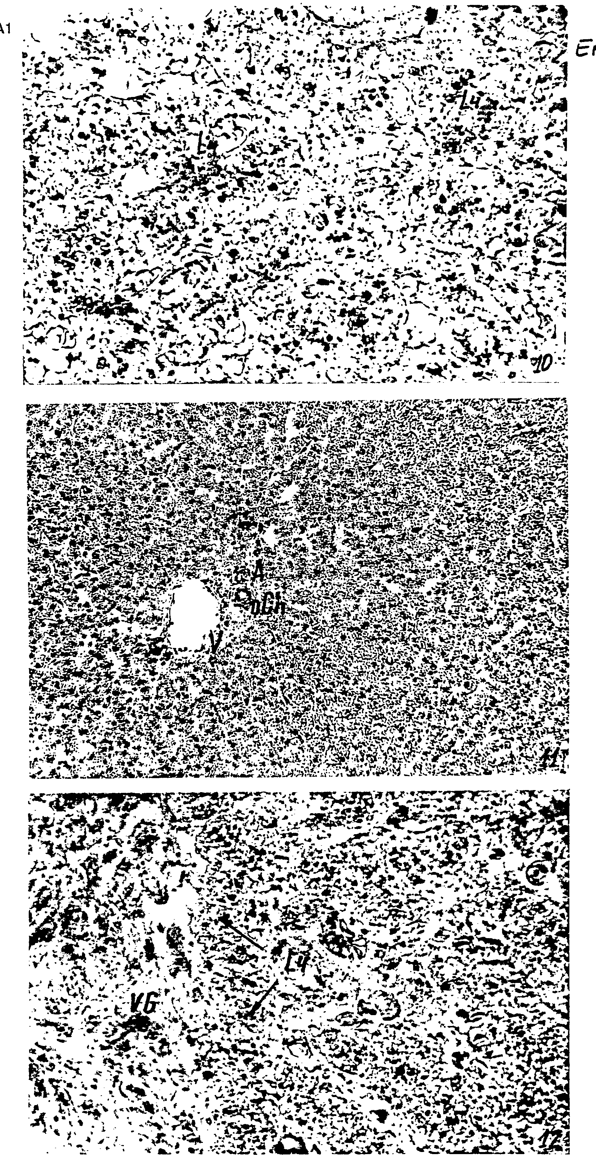

Bei der Untersuchung des Lipidspiegels im Lebergewebe der Kontrolltiere wurde in den Hepatocyten ein normaler Gehalt an Lipidgranulat festgestellt. Die Lipide waren im zentralen und peripheren Bereich der Leber gleichmäßig verteilt (Fig. 1 bis 4). In der Wand des Gallenganges wurden einzelne Lipidgranulae festgestellt, die interlobulär verliefen (Fig. 2).

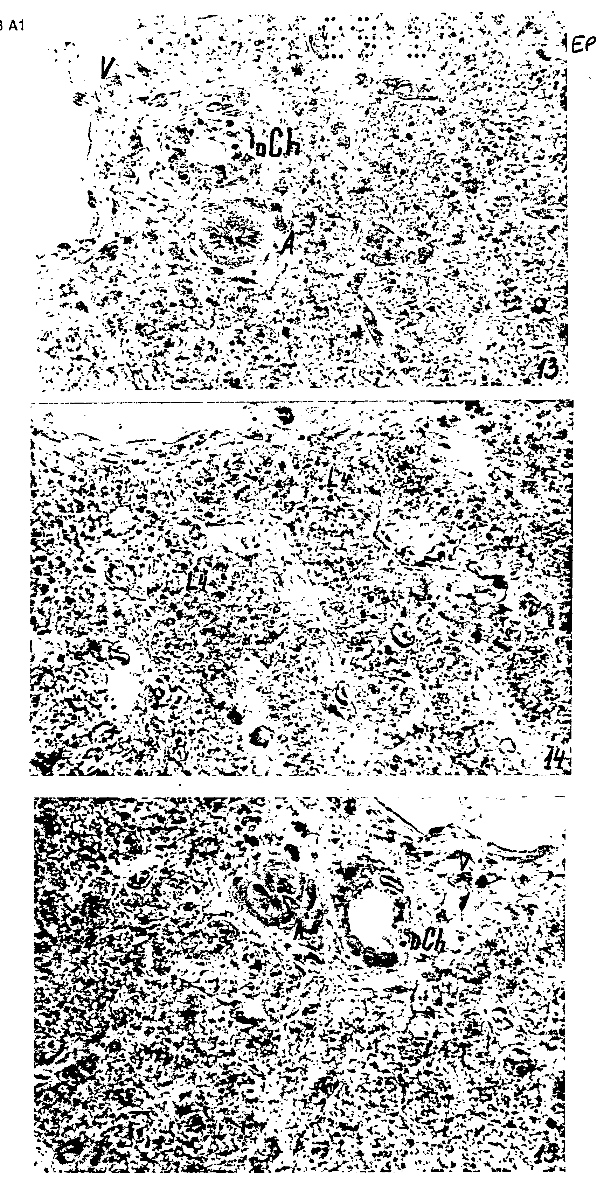

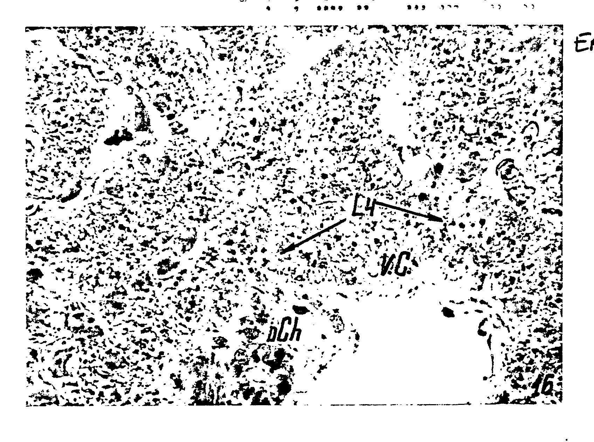

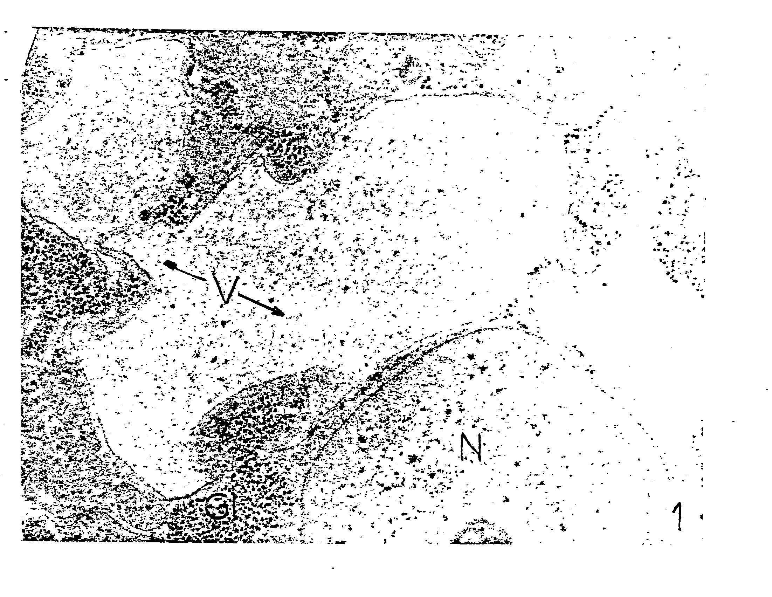

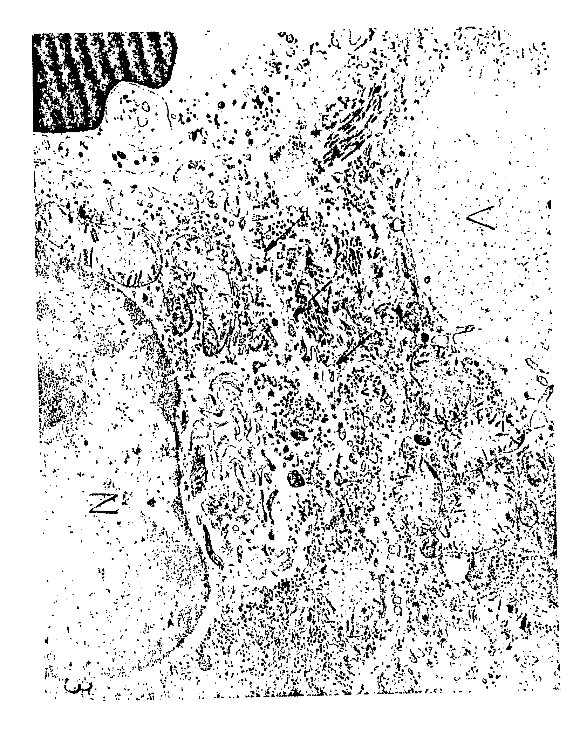

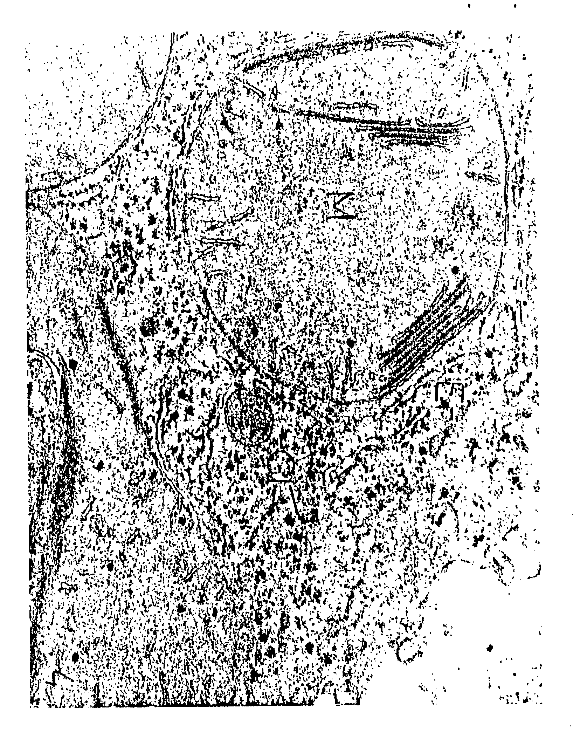

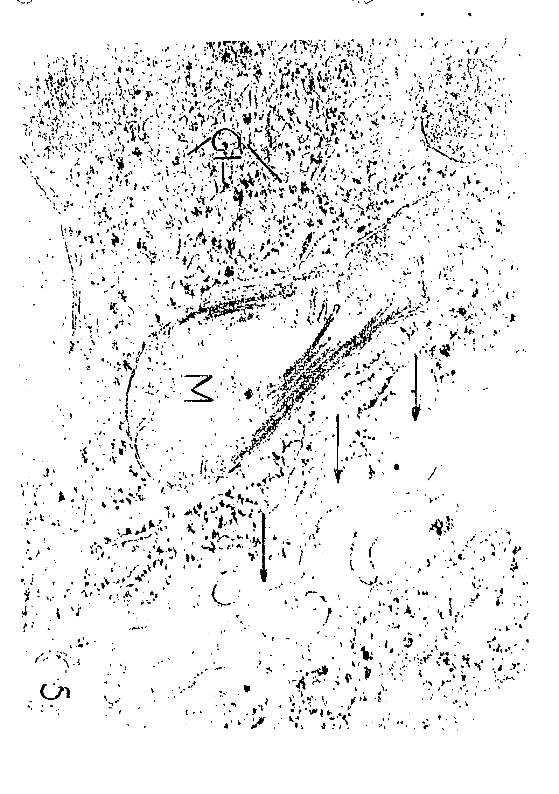

Bei den bloß mit Liposin behandelten Tieren stieg der Lipidspiegel im Lebergewebe stark an. Eine große Menge an Lipidgranulae zeigte sich in den Hepatocyten, wobei diese häufig zu größeren Tropfen zusammengeflossen waren. Die Lipidablagerungen betreffen alle Teile der Leber, und zwar sowohl das Zentrum als auch die Peripherie. Besonders stark angestiegen war die Zahl der Lipidgranulae in den Wandungen der folgenden Blutgefäße: Vena centralis, interlobuläre Verzweigungen der Vena portae und Arteria hepatica, sowie in der Wandung des parallel verlaufenden Gallenganges. Lipidablagerungen zeigten sich vorzugsweise in der Adventitia der Gefäßwandungen (Fig. 5 bis 10).

Die Untersuchungen der Leber der mit Liposin und Orocetam behandelten Tiere ergab einen verringerten Lipidspiegel, der fast dem der normalen Tiere entsprach. Lipidgranulae in den Hepatocyten waren nur vereinzelt festzustellen (Fig. 11 bis 16). Betroffen waren in erster Linie im Zentrum der Leber in der Nähe der Vena centralis angeordnete Hepatocyten (Fig. 12, 14 und 16). Relativ wenig Lipide waren in den Hepatocyten an der Peripherie der Leber enthalten. Die arteriellen und venösen Gefäße waren vollkommen frei von Lipidablagerungen (Fig. 13, 15 und 16). Betroffen war lediglich die Wandung des Gallenganges.

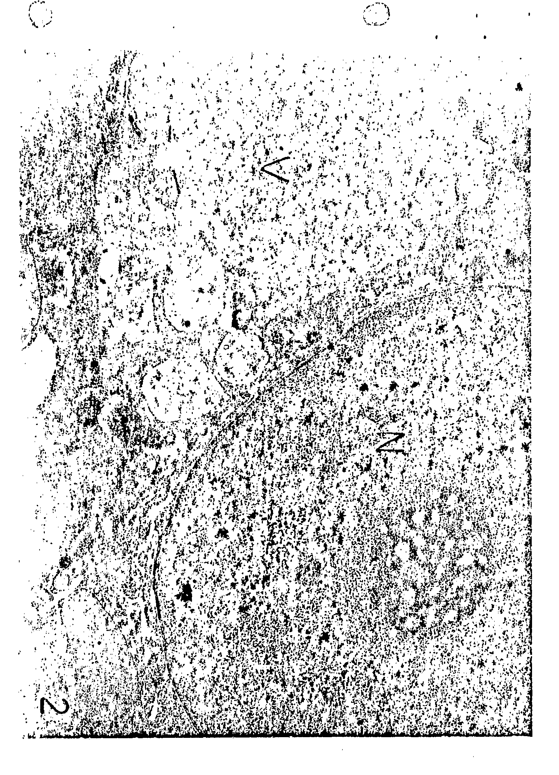

Die Ergebnisse der elektronenmikroskopischen Untersuchungen haben Veräderungen in den membranösen Zellorganellen ergeben. In den untersuchten Hepatocyten der Leber von mit Liposin behandelten Tieren wurden Veränderungen festgestellt, wobei vor allem die membranösen Zellorganellen betroffen waren. Bei den meisten der untersuchten Hepatocyten wurden große Vakuolen festgestellt, die in den meisten Fällen im kernnahen Bereich angesiedelt waren. Wurden in einer Zelle mehrere Vakuolen festgestellt, so standen sie miteinander in Berührung (Fig. 1 und 2). Der Inhalt der Vakuolen war vorzugsweise eine feine globuläre Substanz. In manchen perikapii̟ar angeordneten Häpatocyten waren Teile der Zellmembran entfernt (Fig. 3). Bei den mit ihrer Oberfläche zum Disse-Raum hin orientieren Hepatocyten wurden eine Vielzahl von Mikrovili beobachtet (Fig. 5). Bei allen untersuchten Hepatocyten wurden Lamellen von granuliertem und glattem Reticulum festgestellt. Die Mitochondrien zeigten bei der Mehrzahl der untersuchten Hepatocyten eine veränderte Ultrastruktur und desorientierte Cristae mitochondriales, die in den meisten Fällen gegen die Innenmembran der Mitochondrien entlang der zugehörigen Achse polarisiert waren. Gleichzeitig war die Mitochondrienmatrix stark aufgehellt (Fig. 4, 5 und 6). Sehr häufig stieß man auf Mikrokörperchen mit der dafür kennzeichnenden inneren Kristallstruktur. Die beobachteten Veränderungen in der Dynamik der Mitochondrien zeigen, daß die Mikrokörperchen eine späte Abbauform der Mitochondrien sind (Fig. 6). Die Kerne der beobachteten Hepatocyten blieben intakt.

Sämtliche mit dem Elektronenmikroskop untersuchten Hepatocyten zeigten einen reichen Inhalt an Glykogenkorn. Diese erhöhten die Dichte der Zytoplasmamatrix.

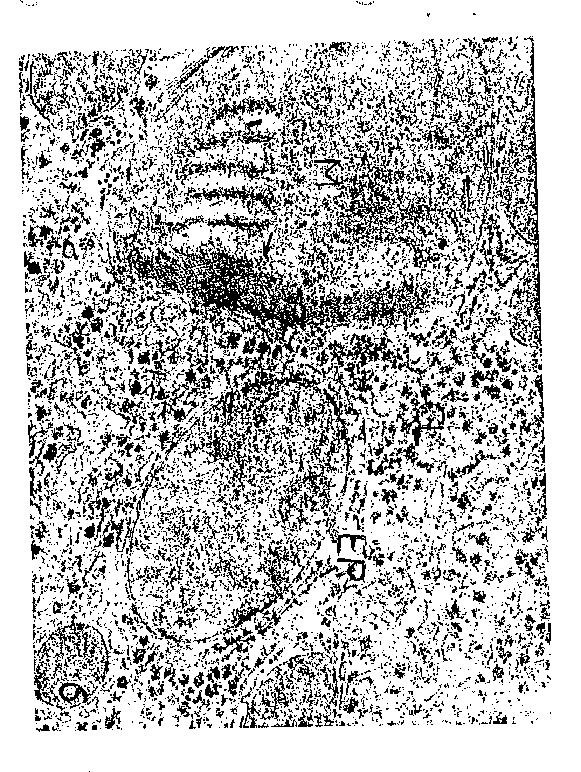

In den Hepatocyten der mit Liposin und Orocetam behandelten Tiere wurden Veränderungen festgestellt, welche eine Rückbildung der oben beschriebenen Veränderungen der Zellorganellen erkennen ließen. Die Kerne mit dem internuklear erweiterten Raum, die entfernten Kernmembranen und die offenen Poren sind morphologischer Ausdruck des aktiven Stoffwechsels zwischen Kern und Zytoplasma (Fig. 7 bis 9). Häufig begegnete man Liposomenstrukturen, die Lipidgranulae enthielten (Fig. 7). In den Makrovaesiculae des Golgiapparats wurden elektronisch dichte Lipoproteidkomplexe festgestellt (Fig. 8). Die Mitochondrien zeigten normale Ultrastruktur, manche von ihnen befanden sich in der Teilungsphase (Fig. 10).

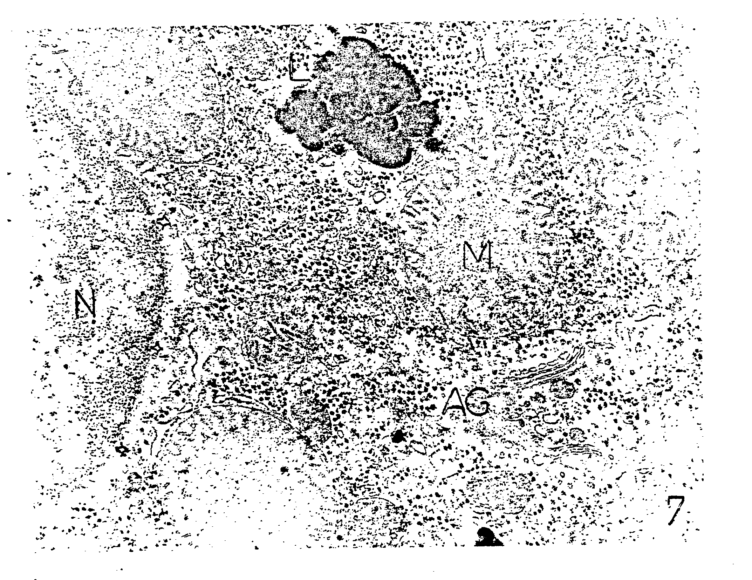

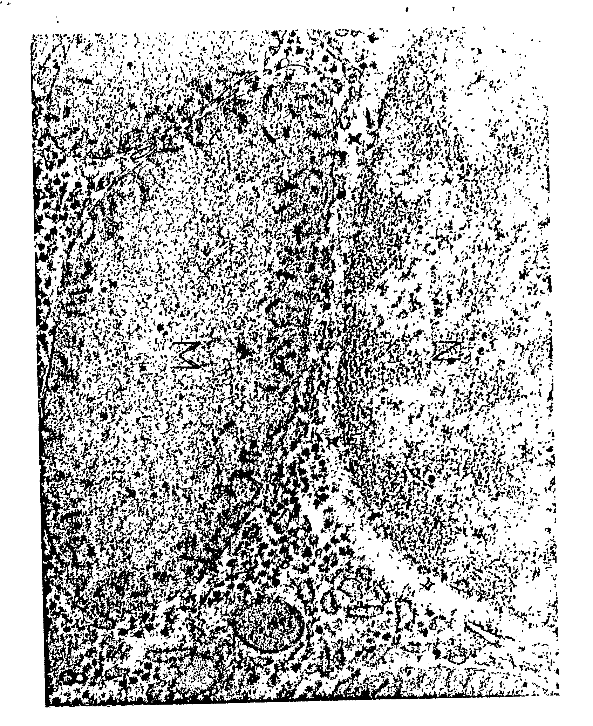

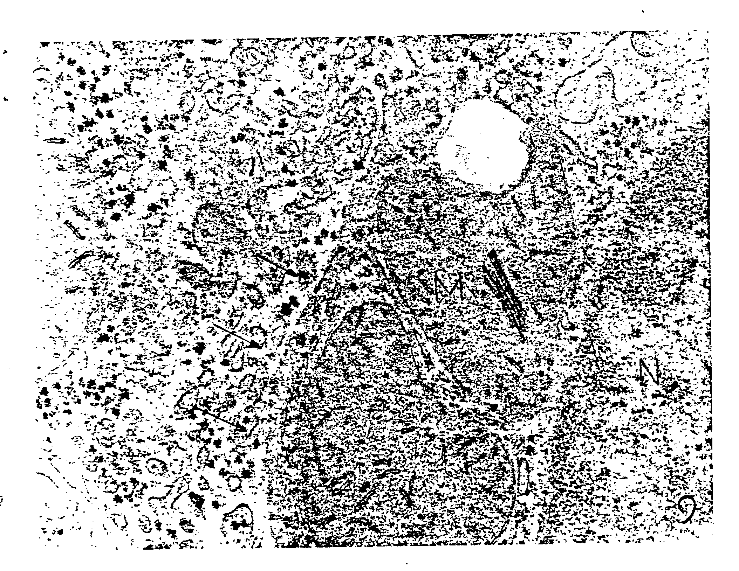

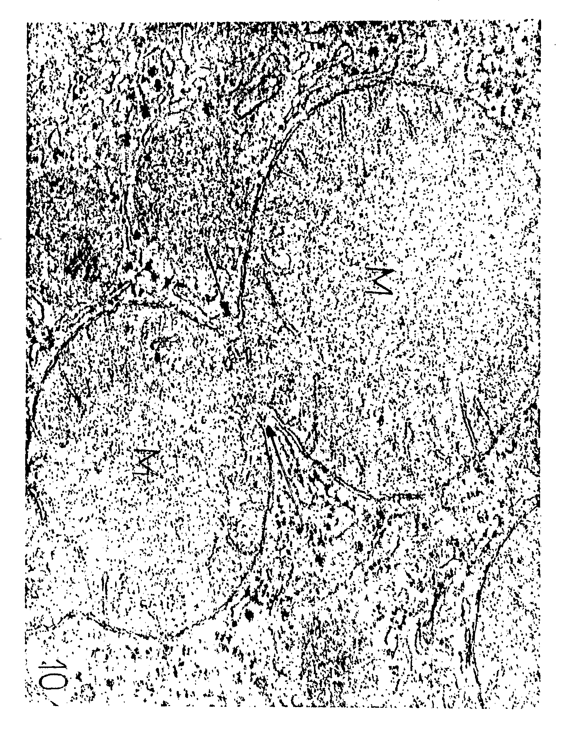

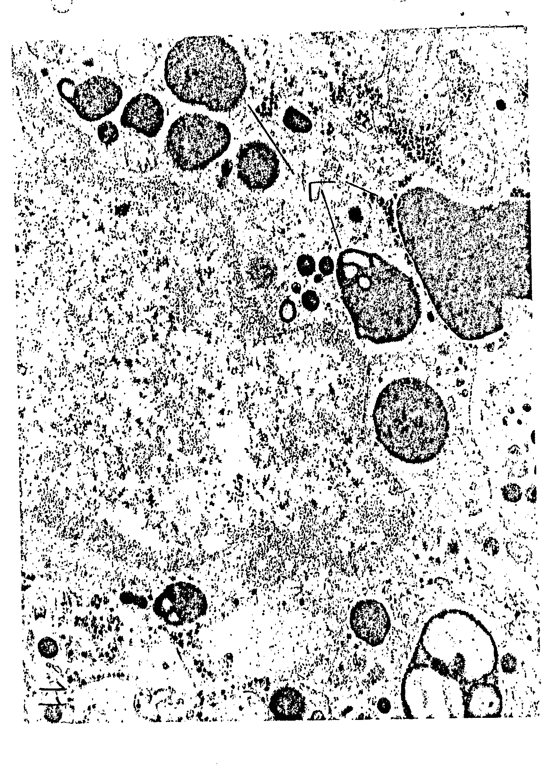

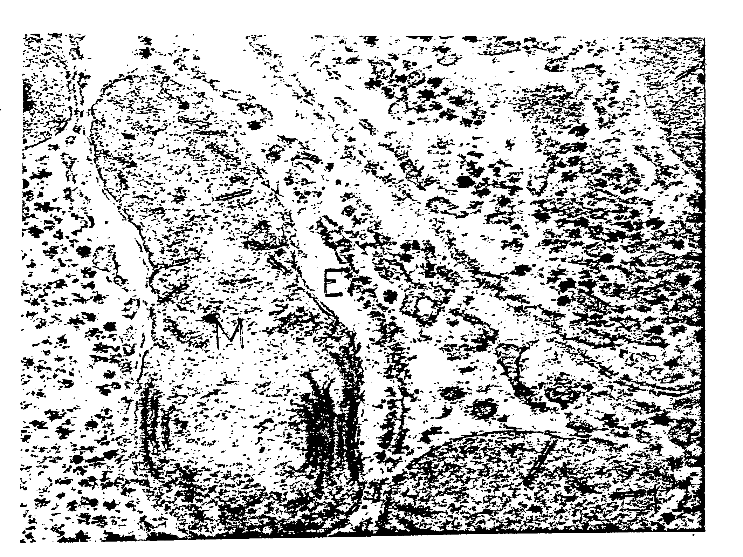

In den Kupfferschen Zellen wurden starke Lipideinschlüsse festgestellt (Fig. 11).

Die Resultate der morphologischen Untersuchungen des Lebergewebes von Tieren, die mit einem Kombinationspräparat aus Liposin und Orocetam behandelt worden waren, ergaben, daß diese Behandlung eindeutig zu einer Verringerung des Lipidspiegels in den Leberzellen und zu einer Wiederherstellung der Ultrastruktur der Hepatocyten führte, was die potenzierende Wirkung des Präparats Orocetam untermauert.

Beispiel 2
====

Ergebnisse der klinischen Tests mit dem erfindungsgemäßen Mittel

Mit Hilfe einer eigens entwickelten EDV-Methode zur Bewertung der Veränderungen auf der leitenden Ebene des vegetativen Nervensystems unter verschiedenen Stressituationen wurde untersucht, ob es bei Infusion der Fettlösung möglicherweise zu einem Abbau der Stresseinwirkung auf dem Organismus kommt, außerdem wurden die Veränderungen im Adaptations-bzw. Regulationsmechanismus bei Einwirkung des Fettpräparats Liposin als solchem oder im Gemisch mit Orocetam untersucht.

Zur Bewertung der Änderung der Stresseinwirkung auf den Organismus und die leitende Ebene des Adaptations- bzw. Regulationssystems wurden die Veränderungen bei der Bildung des Herzrhythmus bei parenteral ernährten Patienten analysiert. Untersucht wurden 4 Patienten: ein Patient nach subtotaler Gastrektomie, der zweite nach korrodierender Verätzung der Speiseröhre durch Salzsäure, der dritte bei vollständiger Stenose der Speiseröhre nach korrodierender Verätzung durch Natronlauge, der auf eine Wiederherstellungsoperation vorbereitet worden war und der vierte nach subtotaler Dünndarmresektion.

Die Untersuchungen wurden in Intervallen von 48 Stunden durchgeführt, wobei bei den ersten beiden Patienten eine Infusion von lediglich Liposin ohne Orocetam verabreicht wurde. Untersuchungsdauer pro Patient: 8 Tage. Die Patienten wurden in zwei Gruppen eingeteilt: Gruppe A mit zwei Untersuchungen bei Infusion von lediglich Liposin und Gruppe B mit zwei Untersuchungen bei Infusion von Liposin und Orocetam. Die Liposininfusion erfolgte nach Verabreichung von 1000 E Heparin bei einer Geschwindigkeit von 30 cal/min. Bei den Patienten der zweiten Gruppe wurden auch 2 Ampullen Orocetam aus einer Flasche mit 10 %-iger Glukose vor und während der Liposininfusion zugetropft.

Die Untersuchungen wurden in Intervallen von 48 Stunden durchgeführt, wobei bei den ersten beiden Patienten eine Fettemulsion von lediglich Liposin ohne Orocetam verabreicht wurde. Untersuchungsdauer pro Patient: 8 Tage. Regelmäßig untersucht wurden die SGOT- und SGPT-Werte, die Alkaliphosphatse und das Lipidprofil. Mit Hilfe der EDV-Methode wurden die Werte folgender Indices festgestellt: Index des vegetativen Gleichgewichts (IVG), Index des adequaten Charakters der peripheren Regulation (IAPR), vegetativer Rhythmusindex (VRI), Spannungsindex (SI), Verdichtungsindex (VI), Pulsfrequenz (PF).

Bei der Untersuchung der Gruppe A ergab die Auswertung der erhaltenen Daten nur geringfügige Abweichungen in den Routineparametern wie Pulsfrequenz, Schwankungen im arteriellen Druck, doppelte Ableitung.

Die Variationsanalyse im R-R-Intervall zeigte eine ansteigende Tendenz bei entsprechendem Anstieg der SI- und VIR-Werte.

Bei der Untersuchung der Gruppe B wurden analoge Veränderungen in den erhaltenen Werten festgestellt, wobei eine raschere Wiederherstellung der Werte der Variationsanalyse nach 12 Stunden festgestellt wurde. Die Veränderungen können als "Streßänderungen" bei beibehaltender Adaptation interpretiert werden. Die Tendenz zur fast vollständigen Wiederherstellung des Ausgangshistogramms wurde in Gruppe A festgestellt und bei der Gruppe B nach 12 Stunden.

Die Ergebnisse der durchgeführten Untersuchungen bestätigen die erhöhte Wirkung der Infusion von Liposin zusammen mit Orocetam oder allein mit Pyracetam oder Orotsäure. Die stabilen Extremwerte der Pulsfrequenz, der Blutdruck und die doppelte Ableitung zeigen die Beibehaltung der Adaptations- bzw. Regulationsmechanismen. Festgestellt wurde eine frühere Wiederherstellung der Werte der Variationsanalyse bei Anwendung von Liposin mit Orocetam.

Die bei jedem Patienten erzielten Ergebnisse sind in den Tabellen 1 bzw. 2 bis 4 zusammengefaßt.

Tabelle 1

| Nr. des Versuchs | PF | | SI | | VIR | | II | | DA | |
|---|---|---|---|---|---|---|---|---|---|---|
| | A | B | A | B | A | B | A | B | A | B |
| 1 | 74 | 78 | 75 | 81 | 9,4 | 10,1 | 115 | 115 | 8510 | 8970 |
| 2 | 82 | 74 | 108 | 94 | 10 | 12 | 120 | 125 | 9840 | 9250 |
| 3 | 76 | 82 | 84 | 91 | 9,6 | 11,4 | 130 | 130 | 9880 | 10660 |
| 4 | 88 | 84 | 107 | 100 | 10,2 | 14 | 110 | 120 | 9680 | 10080 |
| 5 | 90 | 86 | 118 | 114 | 13,2 | 12,8 | 120 | 125 | 10800 | 10750 |
| 6 | 84 | 88 | 184 | 190 | 16,1 | 17,6 | 140 | 140 | 11760 | 12320 |
| 7 | 96 | 90 | 192 | 188 | 16,8 | 15 | 130 | 120 | 12480 | 10800 |
| 8 | 90 | 94 | 180 | 201 | 15,6 | 17,4 | 140 | 130 | 12600 | 12220 |
| arithmetisches Mittel | 85 | 84,5 | 130,6 | 132,4 | 12,6 | 12,7 | 126 | 126 | 10710 | 10647 |

PF = Pulsfrequenz

SI = Streß-Index

VIR = vegetativer Rhythmusindex

II = Intervalle

DA = doppelte Ableitung

A = Patientengruppe, die lediglich Liposininfusionen erhielten;

B = Patientengruppe, die Liposin und Orocetam erhielten

Tabelle 2

| Nr. des Versuchs | PF | | SI | | VIR | | II | | DA | |
|---|---|---|---|---|---|---|---|---|---|---|
| | A | B | A | B | A | B | A | B | A | B |
| 1 | 88 | 90 | 1245 | 1640 | 84 | 86 | 125 | 125 | 11000 | 11250 |
| 2 | 94 | 86 | 640 | 784 | 34 | 35 | 120 | 130 | 11280 | 11180 |
| 3 | 80 | 84 | 324 | 551 | 26 | 37 | 140 | 135 | 11200 | 11340 |
| 4 | 84 | 86 | 472 | 470 | 22,6 | 22 | 110 | 115 | 9240 | 9890 |
| 5 | 102 | 100 | 1840 | 1470 | 76 | 56 | 130 | 120 | 13260 | 12000 |
| 6 | 88 | 86 | 648 | 704 | 48 | 50,5 | 140 | 145 | 12320 | 12470 |
| 7 | 84 | 104 | 1060 | 1740 | 52 | 64 | 125 | 130 | 10500 | 13520 |
| 8 | 108 | 98 | 340 | 280 | 28 | 23,5 | 145 | 140 | 15660 | 13720 |
| arithmetisches Mittel | 91 | 92 | 821,1 | 955 | 46,3 | 46,8 | 129 | 130 | 11709 | 11960 |

PF = Pulsfrequenz

SI = Streß-Index

VIR = vegetativer Rhythmusindex

II = Intervalle

DA = doppelte Ableitung

A = Patientengruppe, die lediglich Liposininfusionen erhielten;

B = Patientengruppe, die Liposin und Orocetam erhielten

Tabelle 3

| Nr. des Versuchs | PF | | SI | | VIR | | II | | DA | |
|---|---|---|---|---|---|---|---|---|---|---|
| | A | B | A | B | A | B | A | B | A | B |
| 1 | 80 | 80 | 456 | 120 | 27,4 | 12,4 | 120 | 120 | 9600 | 9600 |
| 2 | 86 | 78 | 216 | 102 | 12,4 | 12,8 | 120 | 115 | 10320 | 8970 |
| 3 | 80 | 80 | 204 | 108 | 18,4 | 11,8 | 145 | 140 | 11600 | 11200 |
| 4 | 86 | 78 | 184 | 98 | 13,6 | 11,6 | 110 | 115 | 9460 | 8970 |
| 5 | 96 | 92 | 1080 | 211 | 48,4 | 11,4 | 135 | 130 | 12960 | 11960 |
| 6 | 88 | 88 | 143 | 140 | 12,6 | 10,8 | 140 | 140 | 12320 | 12320 |
| 7 | 80 | 84 | 865 | 240 | 39,4 | 13,8 | 130 | 120 | 10400 | 10920 |
| 8 | 100 | 86 | 310 | 270 | 27,3 | 21,4 | 140 | 140 | 14000 | 12040 |
| arithmetisches Mittel | 87 | 83 | 432 | 161 | 25,4 | 13,3 | 130 | 128 | 11310 | 10580 |

PF = Pulsfrequenz

SI = Streß-Index

VIR = vegetativer Rhythmusindex

II = Intervalle

DA = doppelte Ableitung

A = Patientengruppe, die lediglich Liposininfusionen erhielten;

B = Patientengruppe, die Liposin und Orocetam erhielten

Tabelle 4

| Nr. des Versuchs | PF | | SI | | VIR | | II | | DA | |
|---|---|---|---|---|---|---|---|---|---|---|
| | A | B | A | B | A | B | A | B | A | B |
| 1 | 84 | 78 | 82 | 96 | 10,1 | 11,4 | 120 | 120 | 10080 | 9360 |
| 2 | 80 | 76 | 114 | 102 | 12,6 | 11,1 | 125 | 125 | 10000 | 9500 |
| 3 | 76 | 78 | 106 | 90 | 11,8 | 9,4 | 125 | 120 | 9500 | 9360 |
| 4 | 80 | 82 | 91 | 76 | 10,1 | 9,1 | 110 | 115 | 8800 | 9430 |
| 5 | 84 | 86 | 80,4 | 56,4 | 8,9 | 8,1 | 120 | 120 | 10080 | 10320 |
| 6 | 88 | 78 | 104 | 50,4 | 13,4 | 7,8 | 145 | 140 | 12760 | 12320 |
| 7 | 90 | 76 | 132 | 86 | 14,8 | 8,7 | 125 | 125 | 11250 | 9500 |
| 8 | 82 | 80 | 148 | 121 | 12,6 | 12,8 | 145 | 135 | 11890 | 10800 |
| arithmetisches Mittel | 83 | 80 | 107 | 85 | 11,8 | 9,8 | 128 | 125 | 10624 | 10000 |

PF = Pulsfrequenz

SI = Streß-Index

VIR = vegetativer Rhythmusindex

II = Intervalle

DA = doppelte Ableitung

A = Patientengruppe, die lediglich Liposininfusionen erhielten;

B = Patientengruppe, die Liposin und Orocetam erhielten

I Lichtmikroskopie

Histochemischer Nachweis der Lipide im Lebergewebe der Kontrolltiere

Färbung mit Oilared

     1. Zentraler Teil des Leberlappens

Vergrößerung: 150-fach

V.C. - Vena centralis

     2. Interlobulärer Raum, einzelne Lipidgranulae in den Hepatocyten und im Gallengang.

Vergrößerung: 630-fach

V. - Vena portae

A. - Arteria hepatica

dCh - Gallengang

     3. Bereich der Hepatocyten des zentralen Teils des Leberlappens

Vergrößerung: 630-fach

L4 - Lipidgranulae

     4. Hepatocyten des peripheren Teils des Leberlappens.

Vergrößerung: 630-fach

Behandlung mit Liposin

Färbung mit Oilared

5. Lipidingranulae in Bindegewebselementen des interlobulären Raums.

Vergößerung: 150-fach

6. Teil der Wandung der Vena centralis von Lipiden eingenommen, Lipidgranulae in den benachbarten Hepatocyten.

Vergrößerung: 630-fach.

7. Lipidgranulae in Hepatocyten im zentralen Teil des Leberlappens.

Vergrößerung: 630-fach

8. Lipidablagerungen in der Wandung der Gefäße des interlobulären Raums und der Umgebung der Hepatocyten.

Vergrößerung: 630-fach

9. Massive Lipidinfiltrationen in die Wandung der Vena centralis und die angrenzenden Hepatocyten.

Vergrößerung: 630-fach

10. Lipidablagerungen im peripheren Bereich des Leberlappens.

Vergrößerung: 630-fach

Histochemischer Nachweis der Lipide im Lebergewebe von mit Liposin und Orocetam behandelten Tieren

Färbung mit Oilared

11. Interlobulärer Raum mit venösem und arteriellem Gefäß und Gallengang.

Vergrößerung: 150-fach

12. Teil der Wandung der Vena centralis und angrenzende Hepatocyten.

Vergrößerung: 630-fach

13. Interlobulärer Raum, Gefäßtriade, Lipide in der Wandung des Gallengangs.

Vergrößerung: 630-fach

14. Hepatocyten des zentralen Teils des Leberlappens.

Vergrößerung: 630-fach

15. Periphere Bereiche des Leberlappen mit einem Teil der Wandung einer Vene und Gallengang

Vergrößerung: 630-fach

16. Hepatocyten in der Nähe des interlobulären Gefäßes.

Vergrößerung: 630-fach

## II. Elektronenmikroskopie

1. Teil der Hepatocyten des Lebergewebes von mit Liposin behandelten Hunden. Um den Kern angeordnete, in Kontakt stehende Vakuolen (Pfeil). Anhäufung von Glykogengranulae im Zytoplasma

Vergrößerung: 18.200-fach

N - Kern

V - Vakuole

Gl - Glykogen

2. Intakter Hepatocytenkern des Lebergewebes von mit Liposin behandelten Hunden. Feine globuläre Substanz in den Vakuolen.

Vergrößerung: 18.200-fach

3. Entfernung von Bereichen der Zellmembran der Hepatocyten von mit Liposin behandelten Hunden (Pfeile).

Vergrößerung: 15.000-fach

4. Mitochondrien mit veränderter, zur Peripherie hin polarisierter Orientierung der Cristae (M), granuliertes endoplasmatisches Reticulum /ER/, Glykogengranulae (Gl).

Vergrößerung: 52.000-fach

5. Mikrovilöse Oberfläche der Hepatocyten von mit Liposin behandelten Hunden (Pfeile), veränderte Mitochondrien (M).

Vergrößerung: 50.000-fach

6. Sich zu Mikrokörperchen verändernde Mitochondrien und sich bildender kristalliner Kern (M-Pfeile).

Vergrößerung: 61.000-fach

8

7. Teil der Hepatocyten des Lebergewebes von mit Liposin und Orocetam behandelten Hunden. Kernnaher Raum erweitert (N-Sternchen), Golgiapparat mit elektronisch dichten Lipoproteidkomplexen (AG). Lisosom mit Lipideinschlüssen (L).
Vergrößerung: 23.000-fach

8. Erweiterter kernnaher Raum von Hepatocyten des Lebergewebes von mit Liposin und Orocetam behandelten Hunden (N-Sternchen)
Vergrößerung: 39.000

9. Unklare Struktur der Zellmembran von Hepatocyten (Pfeile). Mitochondrien mit tubulösen intramitochondrialen Bildungen und Lipofuscingranulae (M).
Vergrößerung: 39.000-fach

10. Sich teilende Mitochondrien in Hepatocyten des Lebergewebes von mit Liposin und Orocetam behandelten Hunden.
Vergößerung: 54.000-fach

11. Teil einer Kupfferschen Zelle des Lebergewebes von mit Orocetam und Liposin behandelten Hunden. Lisosomenbildungen mit Lipideinschlüssen.
Vergrößerung: 48.000-fach

12. Aufblähung der Mitochondrien und Aufhellung der Matrix Hepatocyten des Lebergewebes von mit Liposin und Orocetam behandelten Hunden.
Vergrößerung: 56.000-fach

## Ansprüche

1. Ernährungstherapeutisches Mittel auf der Basis von Fetten, Eiweißen und/oder Kohlehydraten, dadurch **gekennzeichnet**, daß es nootrope Stoffe enthält und zwar Pyracetam (2-Oxo-1-pyrrolidin-acetamid), Orotsäure oder ein Gemisch beider Stoffe in einem Gewichtsverhältnis des Nahrungsgemisches zum nootropen Stoff von 10:1,1 bis 10:0,05.

2. Mittel nach Punkt 1, dadurch **gekennzeichnet**, daß der nootrope Stoff ein Gemisch von Pyracetam und Orotsäure im Verhältnis 20:1 darstellt.

EP 0 358 008 A1

EPAA-38141

EPAA - 38 141.

EPAR-38141.9

1

EPAA- 38141.9

EPAA-38141.9

EPAA - 38141.9

EP 0 358 008 A1

EPAA - 38141.9

EPAH - 38 141. 9

EP 0 358 008 A1

EPAA-38141.9

EPAA - 38141.9

EPAA - 38141.9

EPAA - 38141.9

EPAA - 38141. 9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 246 177 (G.H. CLARK) <br> * Anspruch 1; Spalte 6, Zeilen 13-18; Spalte 9, Zeilen 25-41; Spalte 12, Zeilen 28-31 * <br> --- | 1 | A 61 K 31/505 <br> A 61 K 31/40 // <br> (A 61 K 31/505 <br> A 61 K 31:40 ) |
| X | BE-A- 643 404 (CHEMOFORMA) <br> * Ansprüche 1,11,14 * <br> --- | 1 | |
| P,X | EP-A-0 278 013 (S O "PHARMACHIM") <br> * Anspruch; Seite 3, Zeilen 35-42; Beispiel 7 * <br> --- | 1 | |
| A | CHEMICAL ABSTRACTS, Band 102, 1985, Seite 44, Zusammenfassung Nr. 179029r, Columbus, Ohio, US; N.N. KARKISHCHENKO et al.: "Comparative study of some parameters of the antidepressant activity of potassium orotate and piracetam", & FARMAKOL. TOKSIKOL. (MOSCOW) 1985, 48(2), 32-5 <br> * Zusammenfassung * <br> ----- | 1,2 | |
| | | | **RECHERCHIERTE SACHGEBIETE (Int. Cl.5)** <br><br> A 61 K |

Der vorliegende Recherchenbericht wurde für alle Patentansprüche erstellt

| Recherchenort | Abschlußdatum der Recherche | Prüfer |
|---|---|---|
| DEN HAAG | 01-12-1989 | PEETERS J.C. |